(19) Europäisches Patentamt
European Patent Office
Office européen des brevets

(11) Publication number: **0 087 319**
**A1**

(12) # EUROPEAN PATENT APPLICATION

(21) Application number: 83300933.5

(22) Date of filing: 23.02.83

(51) Int. Cl.³: C 07 D 223/16
//A61K31/55

(30) Priority: 24.02.82 US 351815

(43) Date of publication of application:
31.08.83 Bulletin 83/35

(84) Designated Contracting States:
AT BE CH DE FR GB IT LI LU NL SE

(71) Applicant: SMITHKLINE BECKMAN CORPORATION
1 Franklin Plaza
Philadelphia Pennsylvania 19101(US)

(72) Inventor: Ackerman, Dennis Marvin
835 Kings Croft
Cherry Hill New Jersey 08034(US)

(72) Inventor: Kaiser, Carl
1105 Sylvan Drive
Haddon Heights New Jersey 08035(US)

(74) Representative: Waters, David Martin, Dr. et al,
Smith Kline & French Laboratories Ltd. Patent
Department Mundells
Welwyn Garden City Hertfordshire AL7 1EY(GB)

(54) **R- and S-isomers of 6-chloro-7,8-dihydroxy-1-(4-hydroxyphenyl)-2,3,4,5-tetrahydro-1H-3-benzazepine.**

(57) Racemic 6-chloro-7,8-dihydroxy-1-(4-hydroxyphenyl)-2,3,4,5-tetrahydro-1H-3-benzazepine has been compared with its R and S enantiomers. The latter have each demonstrated an unexpected pharmacodynamic spectrum applicable to certain abnormal cardiovascular conditions.

EP 0 087 319 A1

# R- AND S-ISOMERS OF 6-CHLORO-7,8-DIHYDROXY-1-(4-HYDROXYPHENYL)-2,3,4,5-TETRAHYDRO-1H-3-BENZAZEPINE

This invention concerns two enantiomers of 6-chloro-7,8-dihydroxy-1-(4-hydroxyphenyl)-2,3,4,5-tetrahydro-1H-3-benzazepine having unexpected spectra of pharmacodynamic activities which are useful in treating certain cardiovascular disorders and a method for preparing them.

A group of 6-halo-7,8-dihydroxy-1-(hydroxyphenyl)-2,3,4,5-tetrahydro-1H-3-benzazepines is described in U.S. Patent No. 4,197,297 to have renal dopaminergic activity which is useful in treating certain clinical conditions such as hypertension and shock. This earlier patent points out the possibility of resolving the diastereoisomers of the chemical compounds disclosed therein as well as the projected biological utilities of the enantiomers. 6-Chloro-7,8-dihydroxy-1-(4-(hydroxyphenyl)-2,3,4,5-tetrahydro-1H-3-benzazepine and its salts were described in the prior art patent to have advantageous pharmacodynamic properties.

We have now found that the separated R and S isomers of 6-chloro-7,8-dihydroxy-1-(4-hydroxyphenyl)-2,3,4,5-tetrahydro-1H-3-benzazepine which differ in configuration at the 1-position of the 1-phenyl-2,3,4,5-tetrahydro-1H-3-benzazepine system have further unexpected pharmacodynamic properties compared with those of their parent racemate or with those of other benzazepine isomers of similar structures. The enantiomers of this invention are represented by the following structural formulas:

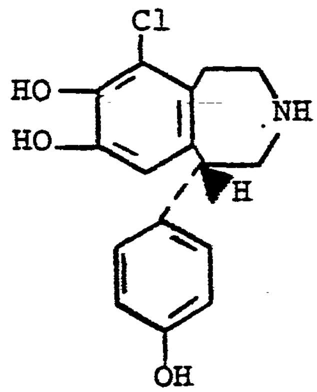

R or d-Isomer (I)

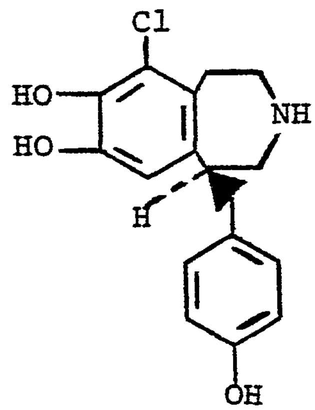

S or l-Isomer (II)

The isomeric compounds (I and II) of this invention include salts, esters, metabolic products such as the possible 6-methyl ether or a sulfate ether as well as any other derivative which may either directly or indirectly owe its biological activity to the parent isomeric compound. Such derivative compounds may be alkanoyl esters of 2-7 carbon atoms, preferably those having the same O-alkanoyl group at all of the 7,8 and 4'-positions. These esters such as the tri-O-isobutyryl, acetyl, butyryl or isoamyl derivatives may be, for example, metabolized in the body to release the active trihydroxy parent isomer.

The pharmaceutically acceptable acid addition salts having the same utility as that of the free bases of Formulas I or II, prepared by methods well known to the art, are formed with both inorganic or organic acids, for example: maleic, fumaric, benzoic, ascorbic, pamoic, succinic, bismethylenesalicylic, methanesulfonic, ethanedisulfonic, acetic, oxalic, propionic, tartaric,

salicylic, citric, gluconic, aspartic, stearic, palmitic, itaconic, glycolic, p-aminobenzoic, glutamic, benzenesulfonic, hydrochloric, hydrobromic, sulfuric, cyclohexylsulfamic, phosphoric and nitric acids. The hydrohalic and especially methanesulfonic acid salts are of particular utility.

It will be understood from this disclosure that each of the R and S isomeric compounds is to be substantially free from the other.

The racemates whose biological activity was reported in U.S. Patent No. 4,197,297 were said there to have a renal dopaminergic mechanism of action which resulted, among other activities, in anti-hypertensive activity due to improved renal function and improved hemodynamics. Due to this mechanism of action, demonstration of hypotensive activity in short term animal models was difficult to achieve.

We have now demonstrated that the separated optical antipodes of certain prior art 7,8-dihydroxy-1-phenyl-2,3,4,5-tetrahydro-1H-3-benzazepines indeed have the predicted spectrum of activities, that is, with most of the biological activity across the board quantitatively residing in one of the isomers.

In contradistinction to the biological properties of the isomers of these prior art compounds, however, the R and S isomers of 6-chloro-7,8-dihydroxy-1-(4-hydroxyphenyl)-2,3,4,5-tetrahydro-1H-3-benzazepine each retain very potent renal dopaminergic activity, from 3-10 times more than that of dopamine itself, while showing a sharp separation of hypotensive activity.

The $ED_{15}$ values in Table A below measure the decrease in vascular resistance in the kidney reflecting dopaminergic activity. They were determined by infusion of the test compound into anesthetized dogs in the procedure described in U.S. Patent No. 4,197,297. It was observed therefore that both the R and S isomers had renal

dopaminergic activities from 3-10 times that of dopamine itself ($ED_{15}$ = 3.5 µg/kg).

The spontaneously hypotensive rat (SHR) data are derived by intraperitoneal administration of the designated dose of the test compound in the following protocol:

Male, Okamoto-Aoki strain spontaneously hypertensive rats (SHR), aged 16-19 weeks, are used for testing. The afternoon before testing, animals are fasted and the following morning the first dose of the test compound is administered along with a 25 ml/kg, p.o. load of normal saline. Animals are then placed, three/cage, in metabolism cages and urine is collected for three hours and subsequently analyzed for sodium, potassium, and creatinine. Indirect systolic blood pressure and heart rate are measured via a tail-cuff method and the animals receive an identical second dose of the test compound. Two hours after the second dose, the systolic blood pressure and heart rate are again determined. Drugs are administered as a solution or suspension in normal saline with 0.02% ascorbic acid.

Table A

| | | | racemate | R | S |
|---|---|---|---|---|---|
| A 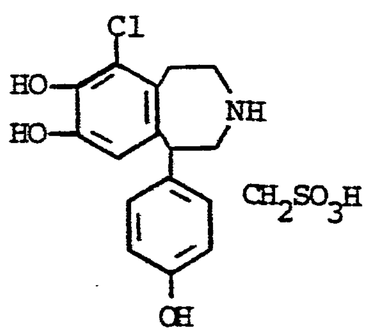 | $ED_{15}$ | (µg/kg) | 0.56 | 0.31 | 0.94 |
| | SHR | (25 mg/kg) | | active | inactive |
| | | (50 mg/kg) | weakly active | active | inactive |
| B 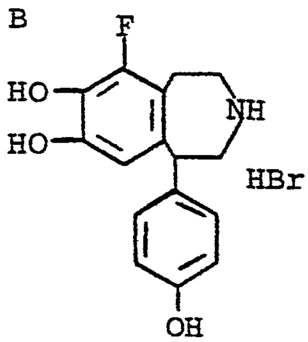 | $ED_{15}$ | | 8.3 | 8.4 | 460 |
| | SHR | (25 mg/kg) | inactive | inactive | inactive |
| | | (50 mg/kg) | inactive | | |

The data demonstrate that the R isomer has a potent hypotensive effect with a strong renal dopaminergic component. The S isomer, by contrast, has a strong renal dopaminergic effect with no significant hypotensive effect. Each therefore has pharmacodynamic properties which are advantageous for specific abnormal cardiovascular conditions.

The separation of hypotensive activities while not substantially separating the renal dopaminergic activity is unexpected. This selectivity of activities was not observed with the closest prior art compound, the 6-fluoro congener, as the data in Table A demonstrate.

In the normal anesthetized dog in which the $ED_{15}$ data were ascertained, the R-isomer, also demonstrated significant lowering of mean arterial blood pressure (MAP) at infusion rates of 9 doses ranging from 0.1 to 810 µg/kg/min. The racemate, on the other hand, demonstrated activity of a lower level and at higher doses. The S-isomer demonstrated only a small decrease in this protocol and then only at the high doses of 90, 270 and 810 µg/kg/min of the range which is considered a minimal true drug effect. Similarly a 24 hour infusion study of the R-isomer in conscious, normotensive rats demonstrated a consistent, more potent hypotensive activity on arterial blood pressure compared with that of the racemate or S-isomer. These results in secondary testing confirm the data presented in Table A.

Both of these isomers would, therefore, have advantages over the racemate in selected abnormal cardiovascular conditions. For example the R isomer would have the ability to treat hypertensive patients with the antihypertensive effects of a potent renal dopaminergic agent combined with a direct hypotensive effect. On the other hand, the S isomer would be most effective in treating cardiovascular abnormalities in which the blood pressure of the patient should not be lowered such as in

hemorrhagic or cardiogenic shock, treating either renal failure or decreased renal function especially in the elderly patient, congestive heart failure, improving cerebral blood flow, hepatic failure, shock lung syndrome, bacterial septicema or any condition in which maintenance of renal blood flow and urine output is desirable.

The pharmaceutical compositions of this invention having the described activity are prepared in conventional dosage unit forms by incorporating a compound of Formulas I or II, a pharmaceutically acceptable acid addition salt or ester deivative thereof, with a nontoxic pharmaceutical carrier according to accepted pharmaceutical procedures in a nontoxic amount sufficient to produce the desired pharmacodynamic activity in a subject, animal or human. Preferably the compositions will contain the active ingredient in an active but nontoxic amount selected from about 25 mg to about 150 mg preferably about 25-100 mg of R isomer per dosage unit but this quantity depends on the route of administration and the conditions of the patient. The S isomer is used in twice the quantities outlined for the R isomer.

The R and S isomers of 6-chloro-7,8-dihydroxy-1-(4-hydroxyphenyl)-2,3,4,5-tetrahydro-1H-3-benzazepine can be prepared by resolution of the racemic compound which is in turn prepared as described in U.S. Patent No. 4,197,297. We have found it convenient and economical to resolve the racemate which is obtained from the ring closure which creates the asymmetric carbon ring member at position 1 of the 2,3,4,5-tetrahydro-1H-3-benzazepine ring system.

Racemic 6-chloro-7,8-dimethoxy-1-(4-methoxyphenyl)-2,3,4,5-tetrahydro-1H-3-benzazepine was reacted with either (-)-dibenzoyl-L-tartaric acid monohydrate or (+)-dibenzoyl-D-tartaric monohydrate to form the crystalline tartrate salts. These salts resolved by fractional recrystallization were treated under alkaline

conditions to obtain the respective resolved bases each of which was then demethylated using standard demethylation agent such as with boron tribromide to give the optically pure R and S isomers of this invention.

We have also found that a minimum of racemization occurs when the demethylation step is carried out using an excess of d,l-methionine in methanesulfonic acid at ambient temperature until the reaction is complete, about 12-64 hours.

The following examples are designed solely to illustrate the isolation and use of the compounds which are the basis of this invention. Temperatures are Centigrade. Other designations of physical constants have significances common in the art.

EXAMPLE 1

A solution of 50 g (0.144 mol) of 6-chloro-7,8-dimethoxy-1-(4-methoxyphenyl)-2,3,4,5-tetrahydro-1*H*-3-benzazepine, 30 ml of 37% formaldehyde (formalin) solution and 17 ml of 88% formic acid was heated at reflux for 10 minutes. The solution was poured onto about 200 ml of ice-water. After the solution was made basic with concentrated aqueous ammonia, the mixture was extracted with ethyl acetate. The extracts were washed with water, dried over magnesium sulfate and concentrated. The residue was recrystallized from petroleum ether to give 46 g (86%) of crystals, 6-chloro-7,8-dimethoxy-3-methyl-1-(4-methoxyphenyl)-2,3,4,5-tetrahydro-1*H*-3-benzazepine; thin layer chromatograph (silica gel, 1:1 hexane:ethyl acetate) gave a single spot $R_f = 0.5$. Under the same conditions the starting material had $R_f = 0.1$. A hydrochloride salt prepared in ethyl acetate melted at 203-205°.

Anal. Calcd. for $C_{20}H_{24}ClNO_3 \cdot HCl$: C, 60.31; H, 6.33; N, 3.52. Found: C, 60.35; H, 6.23; N, 3.49.

A solution of 46 g (0.132 mol) of racemic 6-chloro-7,8-dimethoxy-3-methyl-1-(4-methoxyphenyl)-2,3,4,5-tetrahydro-1H-3-benzazepine and 46 g (0.122 mol) of (-)-dibenzoyl-L-tartaric acid monohydrate in ethanol was diluted with ethyl ether and cooled to give colorless crystals. Recrystallization from methanol gave 21.0 g of colorless needles of the tartrate salt, mp 168° (A).

The combined mother liquors from preparation of the needles (A) were concentrated. The residue was suspended in water and the base was liberated by treatment with aqueous ammonia. Resulting base in ethanol was treated with 30 g (0.08 mol) of (+)-dibenzoyl-D-tartaric acid monohydrate to give 21.0 g of colorless tartrate needles, mp 168° (B).

The above procedure was repeated to give a total of 28 g of both "A" and "B", mp 168°.

Anal. Calcd. for $(C_{20}H_{24}ClNO_3)_2 \cdot C_{18}H_{14}O_8$: C, 63.33; H, 5.86; N, 2.55. Found: C, 62.90; H, 5.63; N, 2.56.

"A" and "B" were each suspended in $H_2O$ and the respective bases were liberated with aqueous ammonia. Bases were extracted with ethyl acetate. The extracts were dried, washed with water, and concentrated to give 20 g (85%) of each isomer. Isomer "A" (an oil) had $[\alpha]_D^{25}$ = -32.7°, -35.4° (c 1, $CH_3OH$). It was converted to a fumarate, mp 175-176°.

Anal. Calcd. for $C_{20}H_{24}ClNO_3 \cdot C_4H_4O_4$: C, 60.31; H, 5.91; N, 2.93. Found: C, 60.38; H, 5.92; N, 2.89.

Isomer "B" had $[\alpha]_D^{25}$ = +36.4°, +34.6° (c 1, $CH_3OH$). The fumarate melted at 176-176°.

Anal. Calcd. for $C_{20}H_{24}ClNO_3 \cdot C_4H_4O_4$: C, 60.31; H, 5.91; N, 2.93. Found: C, 60.61; H, 5.96; N, 2.91.

The methoxy signals in the $CDCl_3$ nuclear magnetic resonance spectrum (NMR) with tris[3-(heptafluoropropylhydroxymethylene)-d-camphorato]europium III were

distinguishable for the isomers and indicated (within the limits of NMR detection) optical purity.

To a solution of 40 g (11.1 mmol) of (-)-6-chloro-7,8-dimethoxy-3-methyl-1-(4-methoxyphenyl)-2,3,4,5-tetrahydro-1H-3-benzazepine (Isomer "A" above) in 100 ml of toluene at $50^{\circ}$ was added 1.4 g (12.9 mmol) of cyanogen bromide in 100 ml of toluene. After standing at $50^{\circ}$ for 1 hour, the solution was filtered and concentrated. The residue was refluxed for 19 hours with a mixture of 60 ml of acetic acid, 140 ml of water, and 17 ml of concentrated hydrochloric acid. After the acidic solution was concentrated, the residue was taken into water and the solution was made alkaline with aqueous ammonia. The mixture was extracted with ethyl acetate. After the extracts were washed with water, they were dried and concentrated to give 3.5 g of crude oil, (-)-6-chloro-7,8-dimethoxy-1-(4-methoxyphenyl)-2,3,4,5-tetrahydro-1H-3-benzazepine. A maleate was recrystallized from acetone-ether to give colorless crystals, m.p. 168.5°, $[\alpha]_D^{25} = -12.0°$ (c 1, $CH_3OH$).

Anal. Calcd. for $C_{19}H_{22}ClNO_3 \cdot C_4H_4O_4$: C, 59.55; H, 5.65; N, 3.02. Found: C, 59.90; H, 5.57; N, 2.90.

Base was liberated from the maleate, 3.1 g (82%), $[\alpha]_D^{25} = -31.3°, -30.2°, -32.1°$ (c 1, $CH_3OH$).

(+)-6-Chloro-7,8-dimethoxy-1-(4-methoxyphenyl)-2,3,4,5-tetrahydro-1H-3-benzazepine was prepared from (+)-6-chloro-7,8-dimethoxy-3-methyl-1-(4-methoxyphenyl)-2,3,4,5-tetrahydro-1H-3-benzazepine (Isomer "B") as described for the (-)-enantiomer; $[\alpha]_D^{25} = +28.9°$, $+32.2°$, $+31.9°$ (c 1, $CH_3OH$). A maleate had mp 168.5°; $[\alpha]_D^{25} = -12.0°$ (c 1, $CH_3OH$).

Anal. Calcd. for $C_{19}H_{22}ClNO_3 \cdot C_4H_4O_4$: C, 59.55; H, 5.65; N, 3.02. Found: C, 59.94; H, 5.57; N, 2.88.

To a solution of 1.6 g (4.6 mmol) of (+)-6-chloro-

7,8-dimethoxy-1-(4-methoxyphenyl)-2,3,4,5-tetrahydro-1H-3-benzazepine in 25 ml of methylene chloride at -10°, under argon, was added 8 ml of a solution of 1 g of boron tribromide in 2.5 ml of methylene chloride (i.e. 3.2 g, 12.8 mmol) [A large excess was used to ascertain if racemization would occur. This excess was not needed]. The solution was allowed to come to 25° during the course of 30 minutes, at which time thin layer analysis indicated the reaction was completed. The reaction was quenched by the addition of excess methanol, concentrated, and the residue was dissolved in 100 ml of warm water. The solution was adjusted to pH 8 with aqueous ammonia. Precipitated solid was filtered and washed with 500 ml of cold water. The solid was slurried in 50 ml of methanol and 98% methanesulfonic acid was added to give pH 1. About 50 ml of ethyl acetate was added to the resulting solution and then ether was added until the solution became cloudy. After cooling, 1.15 g (62%) of colorless crystals of R- or (+)-6-chloro-7,8-dihydroxy-1-(4-hydroxyphenyl)-2,3,4,5-tetrahydro-1H-3-benzazepine methanesulfonate, mp 249-250° (dec), were collected; $[\alpha]_D^{25}$ = +10.2° (c 1, $CH_3OH$).

Anal. Calcd. for $C_{16}H_{16}ClNO_3 \cdot CH_3SO_3H$: C, 50.81; H, 5.02; N, 3.49. Found: C, 50.13; H, 4.99; N, 3.38.

The R-isomer (100 mg) is mixed with 150 mg of lactose and filled into a hard gelatin capsule. These are administered from 3-5 times daily to a hypertensive patient.

This experiment was repeated with a smaller excess of boron tribromide and with S- or (-)-6-chloro-7,8-dimethoxy-1-(4-methoxyphenyl)-2,3,4,5-tetrahydro-1H-3-benzazepine.

EXAMPLE 2

To a solution of 5.0 g (14.3 mmol) of racemic 6-chloro-7,8-dimethoxy-1-(4-methoxyphenyl)-2,3,4,5-tetrahydro-

1H-3-benzazepine prepared as described in U.S. Patent No. 4,197,297 in 50 ml of methanol and 1 ml of water was added 5.4 g (14.3 mmol) of (-)-dibenzoyl-L-tartaric acid monohydrate. After standing at 0$^{o}$ for 18 hours, the crystals that precipitated were collected. The crystalline material was recrystallized from a solution of 50 ml of methanol and 1 ml of water to give 3.5 g of colorless crystals of the dibenzoyl hydrogen tartrate salt of 6-chloro-7,8-dimethoxy-1-(4-methoxyphenyl)-2,3,4,5-tetrahydro-1H-3-benzazepine, mp 155-157°.

Anal. Calcd. for $C_{19}H_{22}ClNO_3 \cdot C_{18}H_{14}O_8$ 1.25 $H_2O$: C, 60.99; H, 5.33; N, 1.92. Found: C, 60.91; H, 5.22; N, 1.90.

All mother liquors were set aside. A suspension of the crystals in water was made alkaline. The mixture was extracted with ethyl acetate, then the extracts were washed with water, dried and concentrated to give 2.2 g (88% isomeric yield) of an oil that slowly crystallized upon standing. IR and NMR spectra indicated this to be the same as the R or (+)-enantiomer of 6-chloro-7,8-dihydroxy-1-(4-methoxyphenyl)-2,3,4,5-tetrahydro-1H-3-benzazepine obtained in Example 1; $[\alpha]_D^{25}$ = +34.7° (c 1, $CH_3OH$). A maleate salt had MP 168.5° and the IR and NMR spectra were identical to that obtained in Example 1.

All the mother liquors from the above separation of the (+)-enantiomer were combined and concentrated. The base was liberated in the usual way and treated with an equivalent amount of (+)-dibenzoyl-D-tartaric acid monohydrate as described above to give 4.0 g of the dibenzoyl hydrogen tartrate salt, mp 155-157°;

Anal. Calcd. for $C_{19}H_{22}ClNO_3 \cdot H_2$: C, 61.37; H, 5.29; N, 1.93. Found: C, 61.35; H, 5.23; N, 1.82. The base was an oil $[\alpha]_D^{25}$ = -32.4° (c 1, $CH_3OH$). IR and NMR were identical with those of the S-base and maleate (mp 168.5°C) obtained in Example 1.

A mixture of 12.5 g (35.9 mmol) of R- or (+)-6-chloro- 7,8-dimethoxy-1-(4-methoxyphenyl)-2,3,4,5-tetrahydro-1H-3-benzazepine, 80.0 g (0.536 mol) of d,l-methionine and 500 ml of 98% methanesulfonic acid was heated gently to afford solution. The solution was stirred at 25° for 64 hours (over a weekend), then it was poured into 1.5 l of ice-water, a trace of ascorbic acid was added, and the pH was adjusted to 8.5 with aqueous ammonia. After being cooled to 0° for 1 hour, the mixture was filtered and the solid was washed with 2 l of cold water. The solid was suspended in 100 ml of methanol and then 98% methanesulfonic acid was added carefully to give pH 5.0. After addition of 200 ml of ethyl acetate and ether (to cloud point), the mixture was cooled. After several hours the crystals were collected (8.2 g, 57%). After three recrystallizations from methanol-ethyl acetate-ether, 7.6 g of colorless crystals of R- or (+)-6-chloro-7,8-dihydroxy-1-(4-hydroxyphenyl)-2,3,4,5-tetrahydro-1H-3-benzazepine methanesulfonate, mp 249-250° (dec), was collected; TLC (silica gel 20:80 $CH_3OH:CHCl_3$) $R_f$ = 0.1 ($R_f$ for starting material is this system, 0.9); $[\alpha]_D^{25}$ = -17.3° (c 1, $H_2O$); $[\alpha]_D^{25}$ = +10.1°, +10.5° (c 1, $CH_3OH$).

Anal. Calcd. for $C_{16}H_{16}ClNO_3 \cdot CH_3SO_3H$: C, 50.81; H, 5.02; N, 3.49. Found: C, 50.52; H, 4.99; N, 3.39.

The R-isomer methanesulfonate (150 mg of base) was mixed with excipients and punched into a scored tablet which was administered as such or broken to a patient in need of antihypertensive therapy from 3-5 times daily.

S- or (-)-6-chloro-7,8-dihydroxy-1-(4-hydroxyphenyl)-2,3,4,5-tetrahydro-1H-3-benzazepine methanesulfonate was prepared from (-)-6-chloro-7,8-dimethoxy-1-(4-methoxyphenyl)-2,3,4,5-tetrahydro-1H-3-benzazepine in the same manner described for the (+)-R-enantiomer in

the preceding experimental procedure. The methanesulfonate had mp 249° (dec); $[\alpha]_D^{25}$ = +16.5° (c 1, $H_2O$); $[\alpha]_D^{25}$ = -10.7°, 10.5° (c 1, $CH_3OH$).

Anal. Calcd. for $C_{16}H_{16}ClNO_3 \cdot CH_3SO_3H$: C, 50.81; H, 5.02; N, 3.49. Found: C, 50.48; H, 4.97; N, 3.42.

The S-isomer methanesulfonate (100 mg of base) was mixed with 150 mg of lactose and filled into a hard gelatin capsule. These are administered four times daily to a patient in need of treatment for the symptoms of congestive heart failure or decreased kidney function.

## Determination of Optical Purity of the R- and S-Enantiomers

A solution of 0.273 g (6.83 mmol) of sodium hydroxide in 10 ml of water was added to 0.5 g (1.25 mmol) of racemic 6-chloro-7,8-dihydroxy-1-(4-hydroxyphenyl)-2,3,4,5-tetrahydro-1H-3-benzazepine methanesulfonate in a minimum amount of water containing a trace of ascorbic acid and under argon. To this mixture was added either 0.345 g (1.37 mmol) of the acid chloride from d, l-α-methoxy-α-trifluoromethylphenylacetic acid or from the l-isomer in 10 ml of tetrahydrofuran. The mixture was stirred for 10 minutes at 25°, then it was adjusted to pH 1 with 2.5 N hydrochloric acid and extracted with ethyl acetate. The organic extracts were washed with water, dried and concentrated to give the respective mandelamides; TLC (silica gel, 20:80 $CH_3OH:CHCl_3$) $R_f$ = 0.9 ($R_f$ of starting material, 0.1); NMR satisfactory.

Analytical high pressure liquid chromatographic separations were performed on 4 mm x 30 cm u-Bondapak $C_{18}$ prepacked columns (Waters Associates) operated at 45°. Elution was carried out using 55% 0.06 M sodium per chlorate adjusted to pH 2.5 with perchloric acid in acetonitrile at a rate of 0.5 ml/min. Samples were applied in acetonitrile adjusted to similarity with the mobile phase. Ultraviolet spectral detection was employed.

The racemic mandelamide subjected to these conditions gave two peaks of equal intensity.

Repetition of the above procedure with the R- and S-enantiomers with the (-)-mandelic acid chloride gave only a single peak corresponding to one of the two observed with the racemate. Integration of the area under the peak indicated >99% optical purity; i.e. >98% enantiomeric excess. This indicates that each R- and S-isomer is substantially free from the other.

EXAMPLE 3

Isobutyryl bromide (1.13 g) is added to a slurry of 1.0 g of R-6-chloro-7,8-dihydroxy-1-(4-hydroxyphenyl)-2,3,4,5-tetrahydro-1<u>H</u>-3-benzazepine hydrobromide in 200 ml of trifluoroacetic acid. The mixture is heated at reflux for 2 hours then stirred for 2 hours. After evaporation to dryness, the residue is taken up in benzene and concentrated to give the desired R-6-chloro-7,8-di-isobutyryloxy-1-(4-isobutyryloxyphenyl)-2,3,4,5-tetrahydro-1<u>H</u>-3-benzazepine hydrobromide.

The tri-isobutyryl derivative of the S-isomer is prepared similarly with isolation as the hydrobromide.

CLAIMS FOR UK, BE, CH, DE, FR, IT, LI, LU, NL AND SE

1. R-6-Chloro-7,8-dihydroxy-1-(4-hydroxyphenyl)-2,3,4,5-tetrahydro-1H-3-benzazepine, which is substantially free from its S-isomer and which is in the form of the free base, its pharmaceutically acceptable acid addition salt or tri-O-lower alkanoyl esters.

2. S-6-Chloro-7,8-dihydroxy-1-(4-hydroxyphenyl)-2,3,4,5-tetrahydro-1H-3-benzazepine, which is substantially free from its R-isomer and which is in the form of the free base, its pharmaceutically acceptable acid addition salts or tri-O-lower alkanoyl esters.

3. R-6-chloro-7,8-dihydroxy-1-(4-hydroxyphenyl)-2,3,4,5-tetrahydro-1H-3-benzazepine.

4. R-6-chloro-7,8-dihydroxy-1-(4-hydroxyphenyl)-2,3,4,5-tetrahydro-1H-3-benzazepine methanesulfonate.

5. S-6-chloro-7,8-dihydroxy-1-(4-hydroxyphenyl)-2,3,4,5-tetrahydro-1H-3-benzazepine.

6. S-6-chloro-7,8-dihydroxy-1-(4-hydroxyphenyl)-2,3,4,5-tetrahydro-1H-3-benzazepine methanesulfonate.

7. The method of preparing either R- or S-6-chloro-7,8-dihydroxy-1-(4-hydroxyphenyl)-2,3,4,5-tetrahyro-1H-3-benzazepine or the salts of either isomer in substantially pure, optically active form comprising reacting the corresponding R- or S- 6-chloro-7,8-dimethoxy-1-(4-methoxyphenyl)-2,3,4,5-tetahydro-1H-3-benzazepine or a salt thereof with boron tribromide and isolating the resulting optically pure R- or S-isomer, respectively, as the base or salt form.

CLAIM FOR AUSTRIA

1. The method of preparing either R- or S- 6-chloro-7,8-dihydroxy-1-(4-hydroxyphenyl)-2,3,4,5-tetrahydro-1H-3-benzazepine or the salts of either isomer in substantially pure, optically active form comprising reacting the corresponding R- or S- 6-chloro-7,8-dimethoxy-1-(4-methoxyphenyl)-2,3,4,5-tetrahydro-1H-3-benzazepine or a salt thereof with boron tribromide and isolating the resulting optically pure R- or S- isomer, respectively, as the base or salt form.

European Patent Office

**0087319**

Application number

# EUROPEAN SEARCH REPORT

EP 83 30 0933

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| D,A | US-A-4 197 297 (J. WEINSTOCK) * Claims 1-8; column 2, lines 57-68 * --- | 1-6 | C 07 D 223/16 // A 61 K 31/55 |
| A | EP-A-0 044 709 (SMITHKLINE CORP.) * Claims 1-5; page 7, lines 17-28 * ----- | 7 | |
| | | | TECHNICAL FIELDS SEARCHED (Int. Cl. 3) |
| | | | C 07 D 223/14 C 07 D 223/16 |

The present search report has been drawn up for all claims

| Place of search | Date of completion of the search | Examiner |
|---|---|---|
| BERLIN | 24-05-1983 | HASS C V F |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82